Europäisches Patentamt

**European Patent Office**

**Office européen des brevets**

(19)

(11) Numéro de publication : **0 267 212 B1**

(12)    **FASCICULE DE BREVET EUROPEEN**

(45) Date de publication du fascicule du brevet :
**10.06.92 Bulletin 92/24**

(51) Int. Cl.$^5$ : **C08B 37/00, C07H 9/02**

(21) Numéro de dépôt : **87902550.0**

(22) Date de dépôt : **21.04.87**

(86) Numéro de dépôt international :
**PCT/FR87/00130**

(87) Numéro de publication internationale :
**WO 87/06592 05.11.87 Gazette 87/24**

(54) **PROCEDE DE PREPARATION, A HAUTE CONCENTRATION DANS LE FLUORURE D'HYDROGENE, D'OLIGO ET POLYOSIDES RAMIFIES NOTAMMENT A PARTIRDE L'AMIDON.**

(30) Priorité : **25.04.86 FR 8606003**

(43) Date de publication de la demande :
**18.05.88 Bulletin 88/20**

(45) Mention de la délivrance du brevet :
**10.06.92 Bulletin 92/24**

(84) Etats contractants désignés :
**BE DE FR GB IT NL**

(56) Documents cités :
**EP-A- 166 362**
**EP-A- 0 051 237**
**EP-A- 0 106 522**
**DE-C- 521 340**
**DE-C- 560 535**
**DE-C- 585 318**
**DE-C- 587 975**
**FR-A- 809 208**

(56) Documents cités :
**Industrial and Engineering Chemistry, vol. 44, No. 5, May 1952, L. Sattler et al.: "Preparation and properties of fructoseand glucose anhydrides", pages 1127-1135, see pages 1133-1135**
**Encyclopedia of Polymer Science (Wiley), vol. 13, page 117, Hemicelluloses**

(73) Titulaire : **BEGHIN-SAY SOCIETE ANONYME**
**F-59239 Thumeries (FR)**

(72) Inventeur : **BOUCHU, Alain**
**11, av. R. Rosselini**
**F-69100 Villeurbanne (FR)**
**Inventeur : CHEDIN, Jean**
**1ter, rue Mornay**
**F-75004 Paris (FR)**
**Inventeur : DEFAYE, Jacques**
**202, chemin du Vercors**
**F-38330 Saint Ismier (FR)**
**Inventeur : WONG, Emile**
**117, cours Jean Jaurès**
**F-38000 Grenoble (FR)**

(74) Mandataire : **David, Daniel et al**
**KAYSERBERG Service Propriété Industrielle**
**23 boulevard Georges Clemenceau**
**F-92402 Courbevoie Cédex (FR)**

EP 0 267 212 B1

# Description

L'invention se rapporte à un procédé de préparation d'oligo- ou polyosides ramifiés solubles dans l'eau, à partir de polyaldosides tels que l'amidon ou la cellulose et concerne en particulier le domaine des produits alimentaires, solubles dans l'eau, susceptibles de présenter une faible valeur calorique parce que difficilement assimilables par l'organisme.

## ETAT DE LA TECHNIQUE

Il est connu que l'action de certains catalyseurs acides sur les monosaccharides et les anhydrosucres, dont la fraction anhydride interne implique au moins le carbone anomérique, tels que le lévoglucosane, ou les unités anhydroglycose constitutives des polyosides, conduit à des oligo- ou polyosides de structures très ramifiées, dont les liaisons sont principalement de type $(1 \rightarrow 6)$ pour les hexoses, leurs dérivés ou les polyaldosides qui les contiennent. (Adv. Carbohydr. Chem. Biochem. 21 (1966) 431-512).

Les catalyseurs les plus couramment utilisés sont les acides minéraux suivants : sulfurique, phosphorique, chlorhydrique, le chlorure de thionyle, ou encore des acides de Lewis. Des acides organiques peuvent également être utilisés.

Les produits obtenus sont généralement constitués d'unités glycopyranose et peuvent présenter une faible proportion d'unités glycofuranose. Dans la plupart des cas, on observe la présence de sous-produits de type furannique, provenant de la désydratation excessive des hexoses ou des pentoses, tels que le 5-hydroxyméthyl 2-furaldéhyde ou le 2-furaldéhyde (E. Huseman et J. Klar, Methods in Carbohydr. Chem. and Biochem. 5, 1965, 180).

Des procédés de fabrication d'oligo- ou polyosides ramifiés basés sur ces résultats ont été décrits :

– Les brevets US 2375564 et 2387275 de la Société CORN PRODUCT REFINING CO décrivent la polymérisation par un traitement à haute température des oses, de préférence à l'état fondu, en présence d'un catalyseur ou d'une combinaison de catalyseurs acides. Dans ce cas, la réaction est conduite à des températures avoisinant 150°C et, sous pression réduite. Les catalyseurs utilisés sont de préférence l'acide chlorhydrique, sulfurique, phosphorique ou borique. Le temps de polymérisation est d'environ 4 à 5 heures. Les produits obtenus sont des mélanges d'oligomères ayant des degrés de polymérisation compris entre 3 et 10.

– Le brevet US 2719179 porte sur la synthèse de polyosides ramifiés à partir de mono- ou oligo-osides en présence d'un catalyseur acide. La méthode est basée sur la déshydratation sous pression réduite ($10^{-5}$ à 100 mm Hg) d'une solution aqueuse de mono- ou oligosaccharides en présence de 0,01 à 5 % d'acide (principalement l'acide chlorhydrique). La température d'évaporation varie entre -80°C et 110°C. Le temps de réaction dure au minimum une minute, mais peut atteindre 24 heures. Les produits sont en général isoles après neutralisation, en solution aqueuse, par le bicarbonate de sodium, suivie d'une précipitation dans un alcool. Le poids moléculaire des produits est estimé compris entre 8100 et 250 000.

– Le brevet US 3766165 de la Société PFIZER INC décrit un procédé de préparation de polyglucosides ou de polymaltosides par polymérisation à l'état fondu de glucose ou de maltose anhydre, en présence d'un acide alimentaire tel que l'acide citrique, comme catalyseur de polymérisation et comme agent de réticulation. La réaction est conduite en présence de 0,1 à 10 % en mole de catalyseur à une température comprise entre 150 et 295°C, sous une pression de $10^{-5}$ à 300 mm Hg. Le temps de réaction dépend de la température du milieu et du taux de réticulation recherché. La réaction est préférentiellement effectuée entre 10 et 180°C en un temps variant de 8 à 24 heures. Par ailleurs, l'addition d'un polyol alimentaire tel que le sorbitol ou le glycérol permet de diminuer la viscosité du mélange réactionnel et d'améliorer la couleur et le goût du produit final. La structure générale des produits est celle d'un polyglucosane lié préférentiellement en $1 \rightarrow 6$, plus ou moins estérifié. Leur masse varie de 1500 à 18000 pour les polyglucoses solubles et de 6000 à 36000 pour les polymères insolubles.

En 1929, Helferich et Böttger ont montré que le traitement de la cellulose avec de l'acide fluorhydrique (HF) conduit à un glucane appelé "Cellane" (Ann., 476 (1929) 150). Z.A. Rogovin et col. mentionnent dans deux publications l'utilisation de HF concentré pour l'hydrolyse de la cellulose et la polycondensation du glucose (Z.A. Rogovin et Yu-L. Pogosov : CA, 53, 22912 h,: Nauchn. Dokl. Vys. Shkoly, Khim. Khim. Tekhnol., (1959), N° 2, p. 368-371 ; Yu-L Pogosov et Z.A. Rogovin, Ca, 55, 24200f,: Uzbekskij Khim. Zh., (1960), p. 58-61). En particulier, ils décrivent la polycondensation du glucose par des solutions aqueuses de HF variant de 45 à 98 %, à des températures de 10 à 30°C. On note qu'ils travaillent avec des concentrations faibles en glucose (10 à 20 %) et obtiennent de petits oligosaccharides de 3 à 5 unités.

Dans la publication parue dans "Industrial and Engineering Chemistry" Vol. 44, N° 5, pages 1127-1135 de mai 1952, on relève que le traitement du D-glucose par le fluorure d'hydrogène, bien qu'à haute concentration de glucose, a conduit à un mélange constitue principalement de produits de départ (taux supérieur à 65 %). Des composes minoritaires (de 25 à 35 %) des "anhydrides du glucose" on pu être détectés, mais leur structure reste relativement mal définie.

Des travaux plus récents (J. Defaye, A. Gadelle, C. Pedersen, Carbohydr. Res. 110 (1982) 217-227) ont montré que le traitement par HF de la cellulose, de l'amylose et du glucose conduit au fluorure de glucopyranosyle. Une évaporation du réactif déplace l'équilibre vers des oligosaccharides de faibles degrés de polymérisation (Dp < 5).

Travaillant sensiblement dans les mêmes conditions de concentration en glucide par rapport à HF, que la publication précitée, la demande de brevet EP 0166362 décrit la préparation d'oligosaccharides de masse comprise entre 180 et 16000 (DP 1 à 100). Les auteurs insistent, dans le cas d'aldoses ou de polyaldosides, sur la nécessité de l'addition d'un polyol tel que le sorbitol, comme dans le brevet US 3766165. Ainsi, la préparation des produits a été réalisée avec une concentration en aldose ou polyaldoside de 10 à 60 % en poids du milieu réactionnel global, et de préférence de 25 à 35 % en présence de 0,01 à 20 % de polyol et de préférence 0,05 à 10 %. La réaction est en général conduite à 25°C pendant environ 1 heure et l'évaporation de la phase liquide sous pression réduite, à une température comprise entre 10 et 80°C, conduit à un sirop. Une étape de neutralisation suivie d'un passage sur résine est généralement nécessaire afin d'éliminer le fluorure d'hydrogène. Les produits obtenus ont une structure ramifiée complexe analogue à celle décrite dans l'article Carbohydr. Res. 110 (1982) 217-227. En revanche, les auteurs mentionnent une masse moléculaire comprise entre 180 et 16000 avec un taux résiduel en glucose de l'ordre de 3 %

Objet de l'invention

L'invention a pour objet la préparation, avec de bons rendements, d'oligo- ou polyosides ramifiés hydrosolubles à partir d'au moins un polyaldoside au moyen d'un traitement simple, dans le fluorure d'hydrogène utilisé comme solvant et réactif.

Conformément à l'invention, le procédé est caractérisé en ce que la concentration de polyaldoside, compté en sec, par rapport au mélange total est au moins égale à 60 %.

La réaction n'est, le plus souvent, pas effectuée en milieu anhydre. Le milieu réactionnel comprend le flurorue d'hydrogène pur accompagné d'une certaine quantité d'eau. Cette dernière peut provenir du HF utilisé, lequel n'est pas nécessairement anhydre, et de l'eau que peut également contenir - en fonction de son séchage plus ou moins poussé - le polyaldoside mis en oeuvre.

Il est bien entendu que le terme fluorure d'hydrogène englobe les cas où, dans le milieu réactionnel, il est associé à une certaine quantité d'eau.

Toutefois la quantité d'eau présente dans le milieu réactionnel ne devrait pas dépasser 40 % de la quantité de fluorure d'hydrogène utilisée, limite au-delà de laquelle la destructuration du polysaccharide traité est partielle.

Les polyaldosides sont des oligo- ou polymères constitues essentiellement de motifs du type aldose. On trouve dans ce groupe l'amidon, l'amylose, la cellulose qui contiennent le motif anhydroglucose comme constituant principal, ou encore le xylane constituant des hemicelluloses, qui contient quant à lui le motif xylose.

Comme cela a été mentionné dans l'état de la technique, il est certes connu qu'un aldose ou un polyaldoside, en solution diluée dans le fluorure d'hyrogène (HF), se transforme principalement en fluorure de glycopyranosyle en équilibre avec une faible proportion de composés secondaires d'autocondensation. Il a été montre également que cet équilibre pouvait être déplacé en faveur des oligomères d'auto-condensation par simple évaporation de HF conduisant à un mélange d'oligomères ramifiés, liés principalement $\alpha$ et $\beta$ (1 → 6) mais également (1 → 2), (1 → 3) et (1 → 4) et de DP < 5 (Carbohydr. Res. 110 (1982) 217-227).

Il a été trouvé, conformément à l'invention, qu'une concentration très élevée de polyaldoside en solution homogène par rapport au fluorure d'hydrogène favorisait le déplacement de l'équilibre dans le milieu vers la formation l'oligomères d'autocondensation avant toute évaporation du solvant, ce qui n'est pas le cas dans les conditions de plus forte dilution par HF. Dans ces conditions, le fluorure de glucopyranosyle, intermédiaire réactif issu de la rupture des liaisons interglycosidiques des polyaldosides, a tendance à fortement réagir, en particulier avec les hydroxyles primaires plus nucléophiles que les autres hydroxyles secondaires constitutifs de l'ose. Il a été trouvé que la grande proportion de glucide dans le milieu n'empêchait pas le fluorure d'hydrogène de jouer à la fois un rôle de solvant, de destructurant des macro- molécules, de réactif et de catalyseur de la réaction, ce qui n'était nullement prévisible. En effet, si on considère la publication mentionnée plus haut de "Industrial and Engineering Chemisty", quine traite que le cas du glucose, on ne peut parvenir à aucun enseignement sur les moyens permettant de ramener le taux du glucose résiduel à la valeur inférieure à 5 %. La quantité minimale de HF nécessaire à la réaction dépend en fait essentiellement des conditions de la dispersion des glucides dans le milieu (agitation, broyage, etc...), de telle façon que cette réaction se déroule en milieu homogène. La réaction est effectuée en milieu liquide ou pâteux homogène dont la viscosité dépend de la concentration des produits. La température et la pression ne sont pas des conditions déterminantes pour la réaction ; elles conditionnent principalement la vitesse à laquelle elle se produit. Ainsi, une élévation de la température de réaction augmente la vitesse de dissolution ainsi que la solubilité des oses dans ce milieu. En conséquence, on préférera opérer à température ambiante, ou assez voisine de l'ambiante, entre 10° et 50°C, et à pression atmosphérique, car c'est finalement la solution la la plus aisée. Elle peut aussi être conduite à une température supérieure dans le cas où cela est reconnu souhaitable, à condition que l'on utilise, bien entendu, les appareils appropriés résistant à la pression et à la corrosion.

Les avantages attendus de cette méthode sont importants au plus industriel. En effet, HF est un produit de manipulation délicate, il est assez coûteux, toxique, très agressif et nécessite un appareillage approprié. On a pu ainsi constater que l'on pouvait obtenir des produits d'autocondensation avec un bon rendement tout en réduisant la quantité de HF nécessaire à la réaction. En conséquence, les problèmes liés à la récupération de l'acide, à son recyclage et aux pertes sont diminués.

Par ailleurs, par rapport aux techniques d'obtention en milieu HF, le procédé à très haute concentration en glucide offre également un mélange réactionnel beaucoup moins corrosif vis-à-vis de l'appareillage utilisé. C'est ainsi qu'il a été observé qu'un mélange amidon - HF à 75 % d'amidon n'attaquait plus que très faiblement la pâte de cellulose par exemple.

Par rapport aux procédés classiques, l'utilisation du fluorure d'hydrogène offre donc l'avantage de température de réaction nettement plus modérées avec diminution des risques de dégradation des produits. Notamment, ce procédé se traduit par une absence de produits de décomposition furannique résultant de la déshydradation.

Pour que la réaction s'effectue dans de bonnes conditions, les polyaldosides sont dissous progressivement dans le fluorure d'hydrogène jusqu'à obtenir la concentration désirée. Le rapport poids de glucide compté en sec / poids du mélange total, peut atteindre une valeur comprise entre 60 % et 80 % et de préférence compris entre 60 et 70 %. Ceci dépend largement des conditions d'agitation mécanique du milieu réactionnel pendant l'ajout progressif du glucide. Le temps de dissolution dépend du glucide utilisé et peut varier de 15 minutes à 2 heures à température ambiante. La solution est alors laissée sous agitation en moyenne pendant 30 minutes.

Lorsque la réaction est terminée, on élimine HF. L'élimination du fluorure d'hydrogène peut être effectuée par balayage avec de l'air sec, ou un gaz inerte, à une température convenable, mais aussi par évaporation sous pression réduite, qui peut être alors menée en 30 minutes environ à des températures comprises entre 0° et 100°C, et de préférence comprises entre 30° et 50°C. L'élimination peut être réalisée par tout autre moyen. Le produit résiduel obtenu parfois sous forme de sirop, mais le plus généralement de poudre sèche, peut renfermer des quantités d'acide non éliminées très variables selon les conditions techniques de l'évaporation.

Le produit, après évaporation, repris par l'eau peut être neutralisé par exemple par le carbonate de calcium (précipitation de $CaF_2$). Le précipité peut être séparé par filtration ou centrifugation. La réduction plus complète des ions fluorure jusqu'à un taux acceptable pour l'alimentation, peut être réalisée par passages sur une colonne de résine échangeuse d'ions mixtes ou selon des méthodes d'électrodialyse ou d'ultrafiltration

Les produits obtenus sont des oligosaccharides ramifies présentant principalement des liaisons de type $\alpha$ , mais aussi $\beta$ (1 → 6), et également une faible proportion de liaisons $\alpha,\beta$ - (1 → 2), (1 → 3) et (1 → 4). Ces produits présentent un taux résiduel de glucose < 3 %, une solubilité de 60 à 75 % dans l'eau, à température ambiante et un taux de non-dialysable de l'ordre de 50 à 75 %.

Ces produits sont nouveaux et constituent également un des objets de l'invention. Le dosage d'extémités réductrices par la méthode au DNS donne un degré de polymérisation moyen de l'ordre de 10. Une chromatographie par exclusion de gel montre que le produit présente un ensemble de masses très polydispersées, et un profil d'élution homogène entre les masses moléculaires 180 et 10 000 (par référence au Dextrane T 10).

Les produits obtenus ont été analyses selon des techniques connues ; elles sont rappelées ci-dessous à titre d'exemple.

Le dosage des extémités réductrices est réalisé par la méthode dite au DNS (acide 2,5 dinitrosalicylique) en prenant pour référence l'unité gentiobiose. Le glucose résiduel a été dosé selon une méthode commercialisée par la société BOEHRINGER. La dialyse est effectuée sous courant d'eau pendant 48 heures dans des tubes à dialyse Visking (commercialisé par Union-Carbide) à paroi mince, retenant des molécules de masse > 6 000.

Pour l'analyse par HPLC on utilise une colonne "Sugar Pak Waters" dans laquelle la phase stationnaire est un gel échangeur d'ions sous forme calcium, cette colonne permet d'évaluer la quantité d'oligomères de haut D.P.. On a utilisé l'analyse par exclusion de gel dont le principe repose sur la rétention sélective des molécules en fonction de leur volume hydrodynamique en solution. Cette technique permet de visualiser la polydispersité et d'apprécier la masse moléculaire moyenne en équivalent dextrine du mélange.

Pour l'analyse par spectrométrie de masse on a utilisé le mode d'ionisation par bombardement d'atomes accélérés (F.A.B.) du composé en solution dans une matrice de glycérol. Les spectres de masse F.A.B. font apparaître des composés de masse 180, 342, 504, 666, etc..., correspondant à la série des oligomères du glucose ayant pour écart de masse une unité anhydroglucose de 162.

En R.M.N. $^{13}$C, trois groupes de signaux peuvent être détectés dans la région des carbones anomériques (90 à 105 p.p.m.) :

a) Le massif centré sur 103,5 p.p.m. correspond aux C-1 des unités β-glucopyranosyle.

b) Le groupe de signaux centré sur 98,5 p.p.m. correspond aux C-1 des unités α-glucopyranosyle. Dans ce massif, les trois signaux principaux à 100,4 p.p.m., 98,6 p.p.m. et 96,7 p.p.m. peuvent être respectivement attribués aux unités glucopyranosyles terminales, liées α (1 → 6) et liées α (1 → 6) α (1 → 2).

Enfin, les deux signaux localisés à 92,9 p.p.m. et 96,7 p.p.m. sont attribués aux carbones des extrémités réductrices.

Par ailleurs, les pics à 61,4 p.p.m. correspondent aux carbones C-6 portant les fonctions hydroxyles libres et à 66,3 p.p.m. aux C-6 liés. Les autres carbones (C-2, C-3, C-4 et C-5) sont détectés dans la région comprise entre 69 et 80 p.p.m..

L'observation des spectres de R.M.N. $^{13}$C confirme bien la structure extrêmement ramifiée de ces composés.

EXEMPLES

Dans les exemples qui suivent, la dissolution du polyaldoside dans HF a été effectuée dans un vase en polyéthylène, sous agitation magnétique. Le mélange dissous, en fin de réaction, est ensuite transféré dans un réacteur inoxydable dans lequel est effectuée l'évaporation sous vide de la plus grande part du liquide - c'est-à-dire HF et un peu d'eau - qui imprègne les produits de la réaction.

Il est bien évident que ces deux phases du procédé, ainsi que la séparation du HF, peuvent être réduites à une phase unique dans le cas où l'on utilise un réacteur doté de moyens d'agitation, ou de broyage, appropriés.

Les rendements indiqués sont des rendements minimaux, étant donné qu'une partie des produits reste fixée sur les parois du vas en polyéthylène où s'effectue la première phase.

Exemple 1

– amidon 75 g (10 % $H_2O$)
– HF 30 g

Dans un vase en polyéthylène, 75 g d'amidon contenant 10 % en poids d'eau sont dissous dans 30 g de fluorure d'hyrogène avec agitation magnétique. L'augmentation de température due à la dissolution de l'amidon est maintenue à 25-30°C par un refroidissement approprié. On introduit d'abord la moitié de l'amidon à engager, puis le reste en 2 fois. Le temps d'addition et de dissolution est au total de 20 mn. On observe une coloration rouge intense du mélange réactionnel.

Ce mélange est ensuite laissé sous agitation magnétique pendant 30 mn à température ambiante puis transféré dans un réacteur en acier inoxydable. L'évaporation sous pression réduite (0,1 mm Hg) du fluorure d'hydrogène est réalisée en 30 mn à 50°C. Le produit obtenu se présente sous la forme d'une pâte épaisse rouge, avec un produit pulvérulent blanc sur les parois du réacteur. Le mélange est repris par le minimum d'eau (100 à 150 ml), puis neutralisé par le carbonate de calcium et filtré. Le filtrat est alors concentré, puis déminéralisé sur résine échangeuse d'ions avant lyophilisation. Il est obtenu sous la forme d'une poudre blanche.

Caracteristiques :

– Produit blanc 57 g (Rdt : 85 %)
– DP moyen estimé par dosage des extrémités réductrices, 10 (unités gentiobiose)
– Teneur résiduelle en glucose, 1,9 %
– Fraction non dialysable, 60 %
– Solubilité dans l'eau à 20°C, 70 %
– Pouvoir rotatoire (α) $^{25}_D$ (c = 1, $H_2O$) + 140

Exemple 2

– Amidon 40 g (1 % $H_2O$)
– HF 20 g

40 g d'amidon préalablement séché (teneur résiduelle en eau 1 %) sont dissous à 30-35°C avec agitation magnétique, en suivant le processus établi dans l'exemple 1. La réaction est laissée 45 mn à température ambiante avec agitation,

L'évaporation du fluorure d'hydrogène est réalisée dans un réacteur en acier inoxydable en 30 mn à 40°C. Le produit obtenu est un solide très cassant qui peut être facilement réduit en poudre. Le taux d'HF résiduel après évaporation est de l'orde de 5 %. Il peut être réduit selon les techniques suivantes :

1) Le mélange est ensuite repris par le minimum d'eau, neutralisé par le carbonate de calcium et filtré. La solution obtenue est ensuite concentrée et déminéralisée par passage sur une résine mixte. L'évaporation à sec conduit à 33 g d'une poudre blanche (Rdt : 83 %)

2) Un simple dégazage du produit brut à l'air libre ou sous un courant d'air sec ou de gaz inerte permet de ramener le taux de fluor à environ 1 %. Le produit obtenu peut alors être mis en solution dans l'eau, puis directement ultrafiltré. Ce traitement peut être complété par passage sur résine échangeuse d'ions, ou par électrodialyse.

Caractéristiques

– DP moyen estimé par dosage des extrémités réductrices, 10 (unités gentiobiose)
– Teneur résiduelle en glucose, 2 %
– Fraction non dialysable, 65 %
– Solubilité dans l'eau à 20°C, 70 %
– Pouvoir rotatoire $(\alpha)^{25}_D$ (c = 1, $H_2O$) + 136

Exemple 3

– Amidon 60 g (10 % $H_2O$)
– HF 20 g

60 g d'amidon (10 % $H_2O$) sont dissous dans 20 g de fluorure d'hydrogène anhydre en 30 mn à 40°C. La réaction est ensuite laissée 1 heure à température ambiante avec agitation.

L'évaporation de fluorure d'hydrogène est réalisée dans un réacteur en acier inoxydable en 30 mn à 60°C. Le produit obtenu, très sirupeux, qui renferme de 8 à 10 % d'HF résiduel, est repris par le minimum d'eau, neutralisé par le carbonate de calcium et filtré. Le filtrat est ensuite traité selon l'exemple 1 et conduit à un produit pulvérulent blanc.

Caractéristiques

– DP moyen estimé par dosage des extrémités réductrices, 10-12 (unités gentiobiose)
– Teneur résiduelle en glucose, 3 %
– Fraction non dialysable, 65 %
– Solubilité dans l'eau à 20°C, 70 %
– Pouvoir rotatoire $(\alpha)^{25}_D$ (c = 1, $H_2O$) + 138

Exemple 4

– Cellulose 40 g (pâte à dissoudre)
– HF, 20 g

Dans un vase en polyéthylène, 20 g de cellulose sont dissous dans le fluorure d'hydrogène en 15 mn à 25°C. On ajoute ensuite les 20 g restants par portions de 5 g en 45 mn à 40°C.

La réaction est ensuite laissée 1 heure avec agitation à 25°C puis évaporée et traitée selon le processus établi dans l'exemple 1. On obtient 34 g d'un produit blanc pulvérulent (Rdt = 85 %).

Caractéristiques

– DP moyen estime par dosage des extrémités réductrices, 10-12 (unités gentiobiose)
– Teneur résiduelle en glucose, 2,5 %
– Fraction non dialysable, 75 %

– Solubilité dans l'eau à 20°C, 65 %
– Pouvoir rotatoire $(\alpha)^{25}_D$ (c = 1, $H_2O$) + 130

**Revendications**

1. Procédé de préparation d'oligo- et polyosides ramifiés, hydrosolubles, par réaction d'au moins un polyaldoside avec le fluorure d'hydrogène puis élimination de l'acide, caractérisé en ce que la réaction est conduite en milieu homogène et en ce que le rapport de polyaldoside, compté en poids sec, au poids du mélange total est compris entre 60 % et 80 %, la proportion d'eau dans le milieu réactionnel étant comprise entre 0 et 40 % de la quantité de fluorure d'hydrogène.

2. Procédé selon la revendication 1, caractérisé en ce que ledit rapport de polyaldoside, compté en poids sec, au poids du mélange total est compris entre 60 et 70 %.

3. Procédé selon l'une des revendications précédentes, caractérisé en ce que ledit polyaldoside est l'amidon, l'amylose, la cellulose, ou le xylane.

4. Procédé selon l'une des revendications précédentes, caractérisé en ce que la réaction est conduite à une température comprise entre 10° et 50°C.

5. Procédé selon l'une des revendications précédentes, caractérisé en ce que l'élimination de l'acide est effectuée par évaporation réalisée à une température comprise entre 0° et 100° C.

**Patentansprüche**

1. Verfahren zur Herstellung von verzweigten, wasserlöslichen Oligo- und Polyosiden durch Reaktion von mindestens einem Polyaldosid mit Fluorwasserstoff und nachfolgender Eliminierung der Säure, dadurch gekennzeichnet, daß die Reaktion in einem homogen Milieu durchgeführt wird, und daß der Anteil des Polyaldosids, berechnet als Trockengewicht, an dem Gewicht der Gesamtmischung zwischen 60 % und 80 % liegt, wobei der Anteil des Wassers in dem Reaktionsmilieu zwischen 0 und 40 % von der Menge des Fluorwasserstoffs beträgt.

2. Verfahren nach Anspruch 1, dadurch gekennzeichnet, daß der Anteil des Polyaldosids, berechnet als Trockengewicht, an dem Gewicht der Gesamtmischung zwischen 60 und 70 % liegt.

3. Verfahren nach einem der vorangehenden Ansprüche, dadurch gekennzeichnet, daß das Polyaldosid Stärke, Amylose, Cellulose oder Xylan ist.

4. Verfahren nach einem der vorangehenden Ansprüche, dadurch gekennzeichnet, daß die Reaktion bei einer Temperatur zwischen 10°C und 50°C durchgeführt wird.

5. Verfahren nach einem der vorangehenden Ansprüche, dadurch gekennzeichnet, daß die Eliminierung der Säure durch eine Evaporation bei einer Temperatur zwischen 0°C und 100°C durchgeführt wird.

## Claims

1. A method of preparing branched water-soluble oligo- and polyosides by reacting at least one polyaldoside with hydrogen fluoride and elimination of the acid, characterised in that the reaction is carried out in a homogeneous medium and the ratio of polyaldoside, by dry weight, to the weight of the total mixture is between 60% and 80%, the proportion of water in the reaction mixture being between 0 and 40% of the quantity of hydrogen fluoride.

2. A method according to claim 1, characterised in that the aforementioned ratio of polyaldoside by dry weight to the weight of the total mixture is between 60 and 70%.

3. A method according to either of the preceding claims, characterised in that the polyaldoside is starch or amylose or cellulose or xylan.

4. A method according to any of the preceding claims, characterised in that the reaction is carried out at a temperature between 10° and 50°C.

5. A method according to any of the preceding claims, characterised in that the acid is eliminated by evaporation at a temperature between 0° and 100°C.